# EUROPÄISCHE PATENTSCHRIFT

(11) **EP 3 837 237 B1**
(45) Veröffentlichungstag und Bekanntmachung des Hinweises auf die Patenterteilung: **24.04.2024**
(21) Anmeldenummer: 19758643.1
(22) Anmeldetag: 14.08.2019
(51) Int. Cl.: C07C 67/10, C08G 64/20

(54) **VERFAHREN ZUR HERSTELLUNG EINES CYCLOALIPHATISCHEN DIESTERS**
METHOD FOR THE PREPARATION OF A CYCLOALIPHATIC DIESTER
PROCÉDÉ DE PRODUCTION D'UN DIESTER CYCLOALIPHATIQUE

(30) Priorität: 17.08.2018 EP 18189636
(43) Veröffentlichungstag der Anmeldung: 23.06.2021
(73) Patentinhaber: Covestro Deutschland AG, 51373 Leverkusen (DE)
(72) Erfinder: MEYER, Alexander, 40489 Düsseldorf (DE); PFINGST, Thomas, 47918 Tönisvorst (DE); HINZMANN, Dirk, 50259 Pulheim (DE)
(74) Vertreter: Levpat
(86) Internationale Anmeldenummer: PCT/EP2019/071779
(87) Internationale Veröffentlichungsnummer: WO 2020/035519

(56) Entgegenhaltungen:
- EP-A1- 3 026 074
- EP-A1- 3 248 999
- WO-A1-02/10111
- JP-A- H07 126 213

## Beschreibung

Die vorliegende Erfindung betrifft ein Verfahren zur Herstellung eines cycloaliphatischen Dieesters, ein Verfahren zur Herstellung eines Polyestercarbonats unter Verwendung eines cycloaliphatischen Dieesters, sowie ein Polyestercarbonat.

Es ist bekannt, dass Polyester, Polycarbonate sowie Polyestercarbonate gute Eigenschaften bezüglich Mechanik, Wärmeformbeständigkeit und Bewitterungsbeständigkeit aufweisen. Jede Polymergruppe weist in Abhängigkeit der verwendeten Monomere bestimmte Schlüsselmerkmale auf, durch die sich derartige Materialien auszeichnen. So weisen Polycarbonate insbesondere gute mechanische Eigenschaften auf, wohingegen Polyester häufig eine bessere Chemikalienbeständigkeit zeigen. Polyestercarbonate zeigen in Abhängigkeit der gewählten Monomere Eigenschaftsprofile aus beiden genannten Gruppen.

Aromatische Polycarbonate oder Polyester weisen zwar häufig ein gutes Eigenschaftsprofil auf, zeigen jedoch bzgl. der Alterungs- und Bewitterungsbeständigkeit Schwächen. So kommt es beispielsweise durch Absorption von UV-Licht zur Vergilbung und ggf. Versprödung dieser thermoplastischen Materialien. Aliphatische Polycarbonate und Polyestercarbonate weisen diesbezüglich bessere Eigenschaften, insbesondere bessere Alterungs- und/oder Bewitterungsbeständigkeiten auf. Ein weiterer Vorteil aliphatischer Polymere ist die bessere Zugänglichkeit der Rohstoffe bzgl. Bioverfügbarkeit. Aliphatische Monomere wie Bernsteinsäure oder Isosorbid sind heute aus Biorohstoffen zugänglich, wohingegen die aromatischen Monomere nicht oder nur beschränkt aus Biorohstoffen zugänglich sind. Im Sinne der vorliegenden Erfindung wird unter dem Ausdruck "biobasiert" verstanden, dass die betreffende chemische Verbindung zum Anmeldezeitpunkt durch einen erneuerbaren und/oder nachwachsenden Rohstoff zugänglich, erhältlich und/oder bevorzugt ein solcher erneuerbarer und/oder nachwachsender Rohstoff ist. Der Ausdruck dient insbesondere der Abgrenzung zu Rohstoffen aus fossilen Rohstoffen. Ob ein Rohstoff biobasiert ist oder auf fossilen Rohstoffen basiert, kann durch die Messung von Kohlenstoffisotopen in dem Rohstoff festgestellt werden, da die relativen Mengen des Kohlenstoffisotops C14 geringer sind in fossilen Rohstoffen. Dies kann beispielsweise gemäß der ASTM D6866-18 (2018) oder der ISO16620-1 bis -5 (2015) erfolgen.

Der Nachteil aliphatischer Polycarbonate oder Polyestercarbonate ist häufig deren geringe Glastemperatur. Deshalb ist es von Vorteil cycloaliphatische Alkohole als (Co)Monomere einzusetzen. Derartige cycloaliphatische Alkohole sind beispielsweise TCD-Alkohol (Tricyclodecandimethanol), Cyclohexandiol, Cyclohexandimethanol und biobasierte Diole auf Basis von 1,4:3,6-Dianhydrohexitolen wie Isosorbid und den Isomeren Isomannid und Iosidid. Um die Glastemperatur weiter zu erhöhen, können auch cycloaliphatische Säuren wie 1,2-, 1,3- oder 1,4-Cyclohexandicarbonsäuren als (Co)Monomere eingesetzt werden. Man erhält dann je nach Wahl der Reaktanden Polyester oder Polyestercarbonate. Die Polyester von Cyclohexandicarbonsäure und Isosorbid sind von Oh et al. in Macromolecules 2013, 46, 2930-2940 beschrieben. Die vorliegende Erfindung ist jedoch bevorzugt auf Polyestercarbonate ausgerichtet.

Polyestercarbonate sind allerdings großtechnisch nicht aus den freien Säuren darstellbar, sondern werden durch Umesterung entsprechender Ester-haltiger Monomere mit Diolen hergestellt. So wird der Polyester aus 1,4-Cylohexandimethanol und 1,4-Cyclohecxandicarbonsäure ausgehend vom Dimethylester der Disäure hergestellt (Blend aus diesem Polyester und Polycarbonat: Xyrex^{®} der DuPont).

Jedoch spielt die Reaktivität dieser Ester-haltigen Monomere von cycloaliphatischen Säuren eine große Rolle bei der späteren Polymerisation mittels Umesterung. Es hat bisher nicht an Versuchen gefehlt, solche cycloaliphatischen Diester bereitzustellen, welche insbesondere für die Herstellung von Polyestercarbonaten geeignet sind. Es ist bekannt, dass derartige Phenylester eine deutlich höhere Reaktivität in Umesterungsreaktionen zeigen als ihre aliphatischen Analoga. Dies ist z.B. in EP 3026074 A1 und in EP 3248999 A1 beschrieben.

In diesen Dokumenten ist auch die Herstellung von Diphenylestern beschrieben. In EP 3026074 A1 wird die direkte Reaktion der Disäure mit Phenol zum entsprechenden Ester beschrieben (Beispiel 1). Jedoch ist die Ausbeute in den Syntheseschritten zum Phenylester eher gering (Beispiel 1; EP3026074). In Beispiel 2 der EP 3026074 A1 wird ein Dimethylester mit Phenol umgesetzt. Auch hier ist die Ausbeute des Diphenylesters verbesserbar.

Beispiel 1 und Beispiel 2 der EP3248999 A1 beschreiben den Einsatz von Lösungsmitteln zur Herstellung des Diphenylesters und erfordern Einsatzstoffe wie Phosgen, welche nicht einfach händelbar sind. Der Einsatz von Lösungsmitteln macht das Verfahren teuer, da die Lösungsmittel nach Abschluss der Reaktion entfernt werden müssen, was weder energetisch vorteilhaft noch aus Umweltgesichtspunkten positiv einzuschätzen ist. Der Einsatz von Phosgen erfordert nicht nur hohe Sicherheitsvorkehrungen sondern ist auch insgesamt teuer. Da die anschließende Reaktion zum aliphatischen Polyestercarbonat ohne Phosgen auskommt, ist die Kombination eines Phosgenverfahrens mit einem Umesterungsverfahren in einem Anlagenteil sehr unvorteilhaft. Damit sind die in EP 3026074 A1 und in EP 3248999 A1 beschriebenen Verfahren nicht optimal.

In der WO2002/10111 A1 wird ein Verfahren zur Herstellung eines aliphatischen Diphenylesters beschrieben, bei dem eine aliphatische, lineare Disäure mit Diphenylcarbonat umgesetzt wird. Hier erfolgt im Anschluss eine Reinigung des Diesters über Umkristallisation unter Verwendung von Lösungsmitteln. Auch diese müssen, wie oben beschrieben, im Anschluss wieder entfernt werden. Zudem enthält dieses Dokument kein Beispiel, bei dem cycloaliphatische Disäuren eingesetzt werden (sondern ausschließlich lineare Disäuren). Im Gegensatz zu linearen Disäuren, sind cycloaliphatische Disäuren meist instabiler. Beispielsweise weist die Thermogravimetrie von 1,4-Cyclohexandicarbonsäure bereits bei 200 °C einen ersten Gewichtsverlust nach. Somit sind die Reaktionsbedingungen von linearen Disäuren nicht ohne Weiteres auf cycloaliphatische Disäuren übertragbar. Insbesondere würde der Fachmann thermische Belastungen von cycloaliphatischen Disäuren vermeiden.

In der JP H07-126213 wird die Reaktion einer aromatischen Disäure wie Terephthalsäure oder Isophtalsäure mit einem Diarylcarbonat beschrieben. Solche aromatischen Disäuren weisen eine sehr hohe thermische Stabilität auf. Aus diesem Grund sind die Reaktionsbedingungen aromatischer Disäuren ebenfalls nicht ohne Weiteres auf cycloaliphatsiche Disäuren übertragbar.

Der vorliegenden Erfindung lag daher die Aufgabe zugrunde ein Verfahren zur Herstellung eines cycloaliphatischen Diesters bereitzustellen, welches mindestens einen Nachteil des Stands der Technik verbessert. Insbesondere lag der vorliegenden Erfindung die Aufgabe zu Grunde ein Verfahren bereitzustellen, bei dem kein Einsatz von Phosgen nötig ist. Zudem soll ein Verfahren bereitgestellt werden, welches lösungsmittelfrei durchgeführt werden kann. Dabei soll der cycloaliphatische Diester jedoch auf einfache Art und Weise ohne hohe Ausbeuteverluste beispielsweise durch thermische Zersetzung und vorzugsweise in hoher Reinheit erhalten werden können.

Mindestens eine, bevorzugt alle der oben genannten Aufgaben wurden durch die vorliegende Erfindung gelöst. Überraschend wurde gefunden, dass die direkte Umsetzung einer cycloaliphatischen Dicarbonsäure mit mindestens einem aliphatischen und/oder aromatischen Carbonat in Anwesenheit eines basischen Katalysators zu dem gewünschten Produkt führt und dass dieses Produkt durch anschließende Destillation in hoher Reinheit und auch hoher Ausbeute erhalten werden kann. Dies war insbesondere überraschend, da nicht davon auszugehen war, dass der cycloaliphatische Dieester ohne Weiteres destillierbar ist. Zudem ist bei einer Destillation das Produkt aus Verweilzeit und Temperatur gegenüber einer Umkristallisation deutlich erhöht. Es war daher nicht davon auszugehen, dass ein cycloaliphatischer Diester den erfindungsgemäßen thermischen Belastungen ohne gravierende Ausbeuteeinbußen übersteht. Vielmehr hätte der Fachmann erwartet, dass die thermische Belastung zum einen zur Zersetzung führt, was die Ausbeute reduziert, und zum anderen damit zu weiteren Nebenprodukten führt, welche das Zielprodukt verunreinigen. Zudem wurde gefunden, dass zur Umesterung die Aktivierung der Dicarbonsäure beispielsweise durch die Darstellung des entsprechenden Säurechlorids nicht nötig ist. Die Herstellung des Säurechlorids erfolgt beispielsweise über Thionylchlorid oder Phosgen - beides sterisch wenig anspruchsvolle sowie hochreaktive Substanzen, welche die oben beschriebenen Nachteile wie den Einsatz von Lösungsmitteln mit sich führen. Dagegen ist ein aliphatisches oder aromatisches Carbonat, wie beispielsweise Diphenylcarbonat, eher reaktionsträge und vor allem im Vergleich zu Phosgen sterisch anspruchsvoller. Da schon der cycloaliphatische Ring die Carbonsäure sterisch schwieriger angreifbar macht - vor allem im Vergleich zu linearen Carbonsäuren - war die Reaktion mit dem ebenfalls sterisch anspruchsvollen Diarylcarbonat höchst überraschend.

Auf diese Weise konnte somit ein Verfahren zur Herstellung eines cycloaliphatischen Dieesters aufgefunden werden, welches keinen Einsatz von Phosgen benötigt und dadurch nicht mit entsprechenden Sicherheitsvorkehrungen verbunden ist. Zudem kann das Verfahren lösungsmittelfrei durchgeführt werden. Dies macht das erfindungsgemäße Verfahren umweltfreundlich. Da eine Destillation zudem relativ unaufwendig ist, kann eine entsprechende Aufarbeitung auch in bereits existierenden Industrieanlagen Anwendung finden. Hier ist zu beachten, dass bei der Reaktion das entstehende Phenol durch Destillation entfernt wird, um das Gleichgewicht der Reaktion zu verschieben. In derselben Apparatur wäre im Anschluss eine Abdestillation des gewünschten Produktes möglich. Damit bietet das erfindungsgemäße Verfahren auch für den Anlagenbau entsprechende Vorteile.

Ein weiterer Vorteil des erfindungsgemäßen Verfahrens ist zudem in der Tatsache zu sehen, dass der cycloaliphatische Diester im Anschluss über einen Schmelzeumesterungsprozess zu einem Polyestercarbonat umgesetzt werden soll. Da diese Umsetzung bereits das Aufschmelzen der Verbindung und damit einem Energieeintrag verbunden ist, kann das abdestillierte Produkt des erfindungsgemäßen Verfahrens direkt diesem Umesterungsprozess zugeführt werden. Hierbei ist dann ein entsprechendes erneutes Aufschmelzen nicht notwendig, da durch die Destillation des cycloaliphatischen Diesters bereits ein entsprechender Energieeintrag erfolgte. Insgesamt kann somit ein unter ökonomischen und ökologischen Aspekten besonders vorteilhaftes Gesamtverfahren, welches die Herstellung des Monomers und die anschließende Polymerisation umfasst, ausgelegt werden.

Zudem hat sich überraschenderweise herausgestellt, dass das Verfahrensprodukt des erfindungsgemäßen Verfahrens ein Monomer liefert, welches zu einem Polyestercarbonat umgesetzt werden kann, welches besonders vorteilhafte Eigenschaften aufweist. Insbesondere wurde gefunden, dass unter Verwendung des erfindungsgemäßen cycloaliphatischen Diesters erfindungsgemäß Polyestercarbonate zugänglich sind, welche eine besonders gute intrinsische Eigenfarbe aufweisen. Diese Farbe ist insbesondere besser gegenüber einem Polyestercarbonat, welches bei einer Reaktion mit einem nicht weiter aufgereinigtem cycloaliphatischen Dieester hergestellt wurde. Daraus ist ersichtlich, dass der Destillationsprozess andere Nebenprodukte und/oder Verunreinigungen aus dem Produkt entfernt als die Umkristallisation.

Erfindungsgemäß wird daher ein Verfahren zur Herstellung eines cycloaliphatischen Diesters der Formel (la) oder (Ib) bereitgestellt, 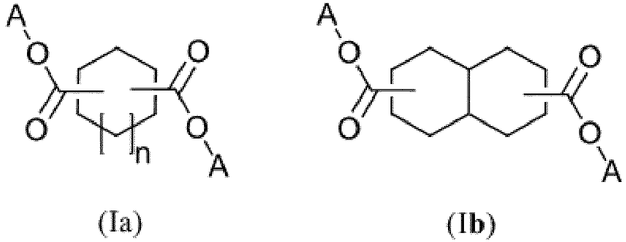 worin
A jeweils unabhängig voneinander für einen aliphatischen oder aromatischen Rest, bevorzugt mit 1 bis 10 Kohlenstoffatomen, besonders bevorzugt einen gegebenenfalls substituierten aromatischen Rest, ganz besonders bevorzugt eine substituierte oder unsubstituierte Phenylgruppe steht
   und
n eine Zahl zwischen 0 und 3, bevorzugt 0 oder 1 ist,
umfassend die Schritte
   (i) Reaktion eines Gemisches, umfassend mindestens eine cycloaliphatische Dicarbonsäure und mindestens ein aliphatisches und/oder aromatisches Carbonat, in Anwesenheit eines basischen Katalysators zu einem cycloaliphatischen Diester der Formeln (la) oder (Ib) und
   (ii) Abtrennung des cycloaliphatischen Diesters der Formel (la) oder (Ib) vom Gemisch des Verfahrensschritts (i) mittels Destillation.

Dabei ist es erfindungsgemäß des Weiteren bevorzugt, dass in der Formel (Ia) oder (Ib) beide A gleich sind. Weiterhin bevorzugt stehen sie für eine substituierte oder unsubstituierte Phenylgruppe, besonders bevorzugt für eine unsubstituierte Phenylgruppe.

Ebenso ist es bevorzugt, dass in der Formel (la) n 0 oder 1 ist.

Schritt (i) des erfindungsgemäßen Verfahrens umfasst die Reaktion eines Gemisches, umfassend mindestens eine cycloaliphatische Dicarbonsäure und mindestens ein aliphatisches und/oder aromatisches Carbonat, in Anwesenheit eines basischen Katalysators zu einem cycloaliphatischen Diester der Formeln (Ia) oder (Ib). Diese Reaktion soll im Folgenden immer wieder beispielhaft an der Reaktion einer Cyclohexandisäure mit Diphenylcarbonat (DPC) erläutert werden. Die Erfindung ist jedoch nicht auf diese speziellen Verbindungen beschränkt, obwohl diese bevorzugt sind. Der Fachmann ist in der Lage, die Reaktion und die entsprechenden Erläuterungen auf andere der beschriebenen Verbindungen zu übertragen.

Es findet in Schritt (i) die folgende (beispielhafte) Reaktion statt:

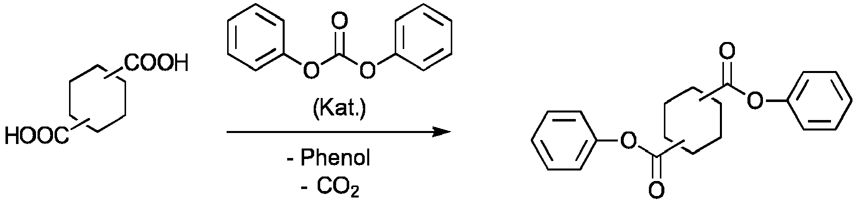

Bei der Reaktion wird somit Kohlenstoffdioxid und Phenol (bzw. eine Verbindung A-OH, wobei A die zu Formel (Ia) und (Ib) beschriebenen Bedeutungen hat) freigesetzt. Es hat sich erfindungsgemäß als vorteilhaft herausgestellt, wenn dieses Kondensationsprodukt A-OH während des Reaktionsschrittes (i) abgetrennt wird. Dabei kann der Ausdruck "während Schritt (i)" bedeuten, dass mit der Abtrennung direkt zu Beginn des Verfahrensschrittes (i) begonnen wird oder aber erst nach einer zeitlichen Verzögerung nach Beginn des Schrittes (i). Die Abtrennung kann auf dem Fachmann bekannte Art und Weise erfolgen, bevorzugt durch Anlegen eines den Reaktionsbedingungen angepassten Unterdrucks. Dabei muss jedoch sichergestellt werden, dass Temperatur und Unterdruck bei Verfahrensschritt (i) so gewählt sind, dass das aliphatische und/oder aromatische Carbonat als Edukt und bevorzugt zunächst auch der cycloaliphatische Diester als Produkt nicht ebenfalls von dem Reaktionsgemisch abgetrennt wird.

Des Weiteren umfasst der erfindungsgemäße Verfahrensschritt (i) bevorzugt mindestens einen, besonders bevorzugt alle der folgenden Schritte (ia) bis (id): (ia) Aufschmelzen der mindestens einen cycloaliphatischen Dicarbonsäure und des aliphatischen und/oder aromatischen Carbonats. Dies erfolgt bevorzugt unter Schutzgasatmosphäre, bevorzugt unter Stickstoff und/oder Argon. Alternativ kann das Aufschmelzen auch im Vakuum erfolgen.

Es ist erfindungsgemäß bevorzugt, dass ein molarer Überschuss an dem mindestens einem aliphatischen und/oder aromatischen Carbonat im Verhältnis zur mindestens einen cycloaliphatischen Dicarbonsäure eingesetzt wird. Besonders bevorzugt wird auf ein Mol der mindestens einen cycloaliphtischen Dicarbonsäure 2,01 bis 2,5, besonders bevorzugt 2,05 bis 2,2 Mole des mindestens einen aliphatischen und/oder aromatischen Carbonats eingesetzt. Dies bringt insbesondere den Vorteil, dass eine hohe Ausbeute erzielt werden kann. Zudem können Verluste am Carbonat, welche gegebenenfalls durch die Abtrennung des Kondensationsproduktes erfolgen, ausgeglichen werden.

In einem Aspekt der Erfindung kann der Schmelze im Verfahrensschritt (ia) ein oder mehrere Stabilisatoren zugesetzt werden. Der erfindungsgemäße cycloaliphatische Diester wird während der Reaktion hohem thermischen Stress ausgesetzt. Zudem ist es bevorzugt, die gesamte Reaktion möglichst frei von Sauerstoff durchzuführen. Sauerstoff führt unweigerlich zur Bildung von Oxidationsprodukten. Um diese Bildung zu minimieren, können ebenfalls Stabilisatoren und/oder Antioxidatien eingesetzt werden.

Bevorzugt wird dieser mindestens eine Stabilisator ausgewählt aus der Gruppe, bestehend aus P-haltigen (Phosphor-haltigen) Stabilisatoren und/oder phenolischen Radikalfängern. Bevorzugt geeignet sind Phosphite und Phosphonite sowie Phosphine. Beispiele sind Triphenylphosphit, Diphenylalkylphosphit, Phenyldialkylphosphit, Tris(nonylphenyl)¬phosphit, Trilaurylphosphit, Trioctadecyl phosphit, Distearyl¬pentaerythritoldiphosphit, Tris(2,4-di-tert-butylphenyl)phosphit, Diisodecylpentaerythritoldiphosphit, Bis(2,4-di-tert-butylphenyl)pentaerythritoldiphosphit, Bis(2,4-di- cumylphenyl)pentaerythritol diphosphit, Bis(2,6-di-tert-butyl-4-methylphenyl)pentaerythritoldiphosphit, Diisodecyloxypentaerythritol¬diphosphit, Bis(2,4-di-tert-butyl-6-methylphenyl)- pentaerythritoldiphosphit, Bis(2,4,6-tris(tert-butylphenyl)pentaerythritoldiphosphit, Tristea-rylsorbitoltriphosphit, Tetrakis(2,4-di-tert-butylphenyl)-4,4'-biphenylen diphosphonit, 6-Isooctyloxy-2,4,8,10-tetra-tert-butyl-12H-dibenz[d,g]-1,3,2-dioxaphosphocin, Bis(2,4-di-tert-butyl-6-methylphenyl)methylphosphit, Bis(2,4-di-tert-butyl-6-methylphenyl)ethylphosphit, 6-Fluoro-2,4,8,10-tetra-tert-butyl-12-methyl-dibenz[d,g]-1 ,3,2-dioxaphosphocin, 2,2',2"-Nitrilo-[triethyltris(3,3',5,5'-tetra-tert-butyl-1,1'-biphenyl-2,2'-diyl)phosphit], 2-Ethylhexyl(3,3',5,5'-tetra-tert-butyl-1,1'-biphenyl-2,2'-diyl)phosphit, 5-Butyl-5-ethyl-2-(2,4,6-tri-tert-butylphenoxy)-1,3,2-dioxaphosphiran, Bis(2,6-di-ter-butyl-4-methylphenyl)pentaerythritol-diphosphit, Triphenylphosphin (TPP), Trialkylphenylphosphin, Bisdiphenylphosphino-ethan oder ein Trinaphthylphosphin. Insbesondere bevorzugt werden Triphenylphosphin (TPP), Irgafos^{®} 168 (Tris(2,4-di-tert-butyl-phenyl)-phosphit) und Tris(nonylphenyl)phosphit oder deren Mischungen eingesetzt.

Ferner können phenolische Radikalfänger wie alkylierte Monophenole, alkylierte Thioalkylphenole, Hydrochinone und alkylierte Hydrochinone eingesetzt werden. Besonders bevorzugt werden Irganox^{®} 1010 (Pentaerythrit- 3-(4-hydroxy-3,5-di-tert-butylphenyl)propionat; CAS: 6683-19-8) und Irganox 1076^{®} (2,6-Di-tert-butyl-4-(octadecanoxycarbonylethyl)-phenol) eingesetzt.

Es ist weiterhin vorteilhaft, eine Kombination von P-haltigen (Phosphor-haltigen) Stabilisator mit phenolischen Radikalfängern einzusetzen. Insbesondere eignet sich die Kombination von (Tris(2,4-di-tert-butyl-phenyl)-phosphit) mit (2,6-Di-tert-butyl-4-(octadecanoxycarbonylethyl)phenol).

(ib) Erhitzen des Gemisches, bevorzugt der aus Schritt (ia) erhaltenen Schmelze. Das Erhitzen erfolgt bevorzugt auf 150 °C bis 300 °C, besonders bevorzugt 180 °C bis 280 °C und insbesondere bevorzugt auf 190 °C bis 240 °C.

(ic) Reaktion des Gemisches, bevorzugt des aus Schritt (ib) erhaltenen Gemisches unter Einbringung von Mischenergie, bevorzugt durch Rühren. Die Reaktionszeit in diesem Schritt hängt von der Menge der Einsatzstoffe ab. Bevorzugt liegt die Reaktionszeit des Schritts (ic) zwischen 0,5 h bis 24 h, bevorzugt zwischen 1 h und 18 h und insbesondere bevorzugt zwischen 1,5 h und 10 h. Dabei ist die Zeit es bevorzugt, die Reaktionszeit so zu wählen, dass das Carbonat annährend vollständig zur Reaktion gebracht wird. Der Fortschritt der Reaktion lässt sich auf dem Fachmann bekannte Weise durch die Bildung von Kohlenstoffdioxid verfolgen (siehe Reaktionsschema oben).

(id) Abtrennung des Kondensationsprodukts A-OH, bevorzugt aus dem aus Schritt (ic) erhaltenen Gemisch. Demgemäß ist es bevorzugt, dass das erfindungsgemäße Verfahren dadurch gekennzeichnet ist, dass während der Reaktion in Verfahrensschritt (i) die flüchtigen Bestandteile, welche einen Siedepunkt unterhalb des cycloaliphatischen Diesters der Formel (Ia) oder (Ib) und unterhalb des aliphatischen und/oder aromatischen Carbonats aufweisen, mittels Destillation gegebenenfalls schrittweise abgetrennt werden. Eine schrittweise Abtrennung wird dabei bevorzugt gewählt, wenn unterschiedliche flüchtige Bestandteile abgetrennt werden. Ebenso bevorzugt wird eine schrittweise Abtrennung gewählt, um eine möglichst vollständige Abtrennung des oder der flüchtigen Bestandteils/e zu gewährleisten. Bei den flüchtigen Bestandteilen handelt es sich bevorzugt um die Verbindung A-OH, wobei

A die zu Formel (Ia) und (Ib) beschriebenen Bedeutungen hat. Bevorzugt handelt es sich dabei um substituiertes oder unsubstituiertes Phenol, wenn A für eine substituierte oder unsubstituierte Phenylgruppe steht.

Die Abtrennung des Kondensationsproduktes erfolgt bevorzugt bei Temperaturen von 150 °C bis 250 °C, besonders bevorzugt 180 °C bis 230 °C. Weiterhin bevorzugt beträgt das Vakuum bei der Abtrennung 500 mbar bis 0,01 mbar. Insbesondere ist es bevorzugt, dass die Abtrennung schrittweise durch Reduktion des Vakuums erfolgt. Ganz besonders bevorzugt beträgt das Vakuum in der letzten Stufe 10 mbar bis 0,01 mbar, um Phenol als Kondensationsprodukt zu entfernen.

Die in Verfahrensschritt (i) eingesetzte mindestens eine cycloaliphatische Dicarbonsäure wird bevorzugt ausgewählt aus einer Verbindung der chemischen Formel (IIa) oder (IIb): 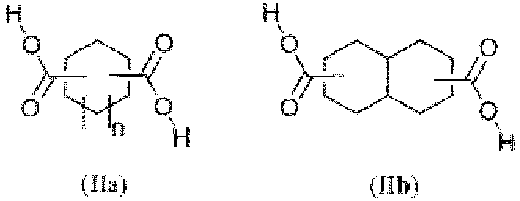 worin
n eine Zahl zwischen 0 und 3 ist. Bevorzugt gelten die für n beschriebenen Bevorzugungen zu Formel (Ia) und (Ib).

Besonders bevorzugt wird die mindestens eine cycloaliphatische Dicarbonsäure in Verfahrensschritt (i) ausgewählt aus der Gruppe, bestehend aus 1,4-Cyclohexandicarbonsäure, 1,3-Cyclohexandicarbonsäure, 1,2-Cyclohexandicarbonsäure, Decahydro-2,4-naphtalindicarbonsäure, Decahydro-2,5-naphtalindicarbonsäure, Decahydro-2,6-naphtalindicarbonsäure, Decahydro-2,7-naphtalindicarbonsäure, ganz besonders bevorzugt aus 1,4-Cyclohexandicarbonsäure, 1,3-Cyclohexandicarbonsäure und 1,2-Cyclohexandicarbonsäure, weiterhin bevorzugt 1,4-Cyclohexandicarbonsäure.

Das in Verfahrensschritt (i) eingesetzte mindestens eine aliphatische und/oder aromatische Carbonat ist bevorzugt mindestens ein aromatisches Carbonat. Cycloaliphatische Disäuren, die mit aliphatischen Alkoholen verestert sind, zeigen in der Umesterungsreaktion zu Polyestercarbonaten eine geringe Reaktivität als solche mit aromatischen Alkoholen, so dass die Molekulargewichte der entsprechenden Polyestercarbonate nach Umesterungsreaktion eher gering sind. Diese Polymere zeigen dann nur ungenügende Eigenschaften. Daher ist der Einsatz von aromatischen Carbonaten besonders vorteilhaft für die resultierenden Polymereigenschaften. Besonders bevorzugt wird in Verfahrensschritt (i) ein aromatisches Carbonat der Formel (2) eingesetzt 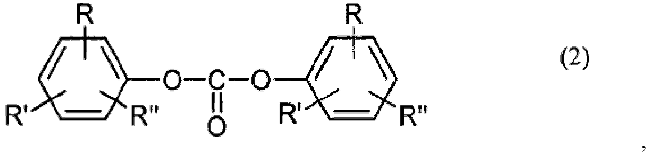 wobei R, R' und R" jeweils unabhängig voneinander gleich oder verschieden sein können und für Wasserstoff, gegebenenfalls verzweigtes C1-C34Alkyl, C7-C34-Alkylaryl oder C6-C34-Aryl stehen, R weiterhin auch -COO-R‴ bedeuten kann, wobei R‴ für gegebenenfalls verzweigtes C1-C34-Alkyl, C7-C34-Alkylaryl oder C6-C34-Aryl steht. Solche Carbonate sind beispielsweise in EP-A 1 609 818 beschrieben. Bevorzugt sind Diphenylcarbonat, 4-tert-Butylphenyl-phenyl-carbonat, Di-(4-tert-butylphenyl)-carbonat, Biphenyl-4-yl-phenyl-carbonat, Di-(biphenyl-4-yl)-carbonat, 4-(1 -Methyl-1 -phenylethyl)-phenyl-phenyl-carbonat und Di-[4-(1 -methyl-1 -phenylethyl)-phenyl]-carbonat. Ganz besonders bevorzugt wird in Verfahrensschritt (i) substituiertes oder unsubstituiertes, bevorzugt unsubstituiertes Diphenylcarbonat als aromatisches Carbonat eingesetzt.

Die Carbonate können auch mit Restgehalten der Monohydroxyarylverbindungen, aus denen sie hergestellt wurden, eingesetzt werden. Die Restgehalte der Monohydroxyarylverbindungen können bis zu 20%, vorzugsweise 10%, besonders bevorzugt bis 5% und ganz besonders bevorzugt bis zu 2% betragen. Dies bedeutet, dass im erfindungsgemäßen Verfahren auch Carbonate verwendet werden können, welche keine aufwendigen Reinigung nach ihrem Herstellungsverfahren unterzogen werden müssen. Da im erfindungsgemäßen Verfahren die Monohydroxyarylverbindung, aus der das Carbonat erhalten wurde, als Kondensationsprodukt A-OH wieder anfällt und bevorzugt abgetrennt wird, stören diese Verunreinigungen die Reaktion nicht. Durch ein so günstig hergestelltes Carbonat kann das erfindungsgemäße Verfahren insgesamt noch wirtschaftlich vorteilhafter gestaltet werden.

In einem anderen Aspekt kann das mindestens eine aliphatische und/oder aromatische Carbonat auch phosgenfrei hergestellt werden. Dies ermöglicht es, dass das gesamte Verfahren zur Herstellung eines Polyestercarbonats phosgenfrei durchgeführt werden kann.

Es hat sich herausgestellt, dass erfindungsgemäß die Anwesenheit mindestens eines basischen Katalysators im Verfahrensschritt (i) notwendig ist. Andernfalls findet die Reaktion zwischen Carbonat und Disäure nicht statt. Dies belegt, dass Reaktionsbedingungen in Bezug auf lineare aliphatische Dicarbonsäure nicht auf die Umsetzung von cycloaliphatischen Dicarbonsäuren übertragen werden können, da in der WO 02/10111 A1 die Reaktion von DPC mit linearen aliphatischen Dicarbonsäuren bei Temperaturen von 180 °C beschrieben wird und auch bevorzugt ist. Es wird beschrieben, dass Reaktionsprodukte, welche unter Anwesenheit eines basischen Katalysators erhalten werden, mehr Verunreinigungen aufweisen. Erfindungsgemäß wurde nun gefunden, dass bei der Reaktion mit cycloaliphatischen Dicarbonsäuren ein Katalysator zumindest bei moderaten Temperaturen notwendig ist und dass dennoch mit dem erfindungsgemäßen Verfahren Produkte mit hoher Reinheit erhalten werden können.

Bei dem basischen Katalysator handelt es sich um eine Base oder einen basischen Umesterungskatalysator. Hierbei können beispielsweise folgende Basen bzw. Katalysatoren eingesetzt werden:
Alkalimetallverbindungen wie LiOH, NaOH, KOH, CsOH, Li₂CO₃, Na₂CO₃, K₂CO₃, Cs₂CO₃, LiOAc, NaOAc, KOAc, CsOAc, Eralkalimetallverbindungen wie Ca(OH)₂, Ba(OH)₂, Mg(OH)₂, Sr(OH)₂, CaCO₃, BaCO₃, MgCO₃, SrCO₃, Ca(OAc)₂, Ba(OAc)₂, Mg(OAc)₂, Sr(OAc)₂, anorganische oder organische basische Verbindungen beispielsweise Lithium-, Natrium-, Kalium-, Cäsium-, Calzium-, Barium-, Magnesium-, -halogenide, -phenolate (wie Na-phenolat), -diphenolate, -fluoride, -phosphate, -hydrogenphosphate, -boranate, Stickstoff- und Phosphorbasen wie beispielsweise Tetramethylammoniumhydroxid, Tetramethylammoniumacetat, Tetramethylammoniumfluorid, Tetramethylammoniumtetraphenylboranat, Tetraphenylphosphoniumfluorid, Tetraphenylphosphoniumtetraphenylboranat, Dimethyldiphenylammoniumhydoxid, Tetraethylammoniumhydroxid, DBU, DBN oder Guanidinsysteme wie beispielsweise das 1,5,7-Triazabicyclo-[4,4,0]-dec-5-en, 7-Phenyl-1,5,7-triazabicyclo-[4,4,0]-dec-5-en, 7-Methyl-1,5,7-triazabicyclo-[4,4,0]-dec-5-en, 7,7'-Hexylidendi-1,5,7-triazabi-cyclo-[4,4,0]-dec-5-en, 7,7'-Decylidendi-1,5,7-triazabicyclo-[4,4,0]-dec-5-en, 7,7'-Dodecyliden-di-1,5,7-tri-aza-bicyclo-[4,4,0]-dec-5-en oder Phosphazene wie bei-spielsweise die Phosphazen-Base P1-t-Oct = tert.-Octyl-imino-tris-(dimethylamino)-phosphoran, Phosphazen-Base P1-t-Butyl = tert.-Butyl-imino-tris-(dimethylamino)-phosphoran, BEMP = 2-tert.-Butylimino-2-diethylamino-1,3-dimethyl-perhydro-1,3,2-diaza-2-phos-phoran. Ferner Tetramethylammoniumhydroxid, Tetramethylammoniumacetat, Tetramethylammoniumfluorid, Tetramethylammoniumtetraphenylboranat, Dimethyldiphenylammoniumhydroxid, Tetraethylammoniumhydroxid,

Cethyltrimethylammoniumtetraphenylboranat und Cethyltrimethylammoniumphenolat. Geeignet sind ferner Phosphoniumkatalysatoren der Formel (8): 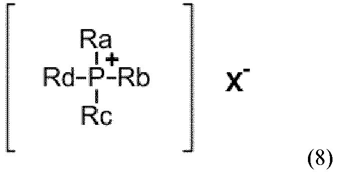 wobei Ra, Rb, Rc und Rd dieselben oder verschiedene C1-C10-Alkyle, C6-C14-Aryle, C7-C15-Arylalkyle oder C5-C6-Cycloalkyle, bevorzugt Methyl oder C6-C14-Aryle, besonders bevorzugt Methyl oder Phenyl sein können, und X- ein Anion wie Hydroxyd, Sulfat, Hydrogensulfat, Hydrogencarbonat, Carbonat oder ein Halogenid, bevorzugt Chlorid oder ein Alkylat bzw. Arylat der Formel -OR sein kann, wobei Rein C6-C14-Aryl, C7-C15-Arylalkyl oder C5-C6-Cycloalkyl, bevorzugt Phenyl sein kann. Ferner können Umesterungskatalysatoren wie Titanalkanolate wie Titantetrabutanolat, tertiäre Amine wie Triethylamin, DMF, Dimethylsacetamid, Methylpyrrolidon, Tetramethylharnstoff, Dimethylimidazolidon hexalalkylguanidium-halogene. Ferner AlCl₃, FeCl₃, BiCl₃, GaCl₃,SbCl₅, BF₃, Bi(OTf)₃, TiCl₄, ZrCl₄, TiB₄ oder ZrBr₄. Bevorzugte Katalysatoren sind Tetraphenylphosphoniumchlorid, Tetraphenylphosphoniumhydroxid, Tetraphenylphosphoniumphenolat, Natriumphenolat und 4-Dimethylaminpyridin; von diesen besonders bevorzugt ist Tetraphenylphosphoniumphenolat. Die Katalysatoren können auch in beliebigen Kombination (zwei oder mehrere) miteinander eingesetzt werden. Insbesondere können zusätzlich auch Cokatalysatoren eingesetzt werden, um die Geschwindigkeit der Umesterung zu erhöhen. Dazu gehören beispielsweise die vorstehend genannten alkalisch wirkenden Salze der Alkalimetalle und/oder Erdalkalimetalle.

Diese Katalysatoren werden bevorzugt in Mengen von 10⁻² bis 10⁻⁸ Mol, bezogen auf 1 Mol der cycloaliphatischen Dicarbonsäure, eingesetzt. Die Mengen der alkalisch wirkenden Salze als Cokatalysator können im Bereich von 1 bis 500 ppb, vorzugsweise 5 bis 300 ppb und besonders bevorzugt 5 bis 200 ppb, bezogen auf 1 Mol der cycloaliphatischen Dicarbonsäure, betragen.

Bevorzugt wird die Reaktion des Verfahrensschritts (i) bei Temperaturen von 180 bis 280 °C durchgeführt wird. Dabei wird diese Temperatur bevorzugt in den Verfahrensschritten (ib) bis (id), falls vorhanden, eingestellt bzw. beibehalten.

Im erfindungsgemäßen Verfahrensschritt (ii) erfolgt die Abtrennung des cycloaliphatischen Diesters der Formel (Ia) oder (Ib) vom Gemisch des Verfahrensschritts (i) mittels Destillation.

Der Begriff der "Destillation" ist dem Fachmann als ein thermisches Trennverfahren an sich bekannt. Die Trennwirkung der Destillation beruht auf der Ungleichverteilung der Komponenten auf die Gas- und Flüssigkeitsphase bei eingestelltem thermodynamischen Gleichgewicht. Die Destillation ist besonders dann geeignet, wenn sich die Siedepunkte der zu trennenden Flüssigkeiten unterscheiden bzw. wenn diese einen unterschiedlichen Dampfdruck bei gleicher Temperatur aufweisen. Dem Fachmann ist bekannt, wie er eine solche Trennung über Destillation durchführt und wie er die entsprechenden Apparate unter Berücksichtigung der Abweichungen vom idealen thermodynamischen Gleichgewicht auszuführen hat.

Insbesondere werden unter dem Begriff Destillation die Verfahren der Rektifikation, fraktionierten Destillation, Vakuumdestillation oder Schleppdestillation und beliebige Kombinationen dieser Verfahren oder mit weiteren thermischen Trennverfahren, beispielsweise der Adsorptions-/Desorptionsverfahren und des Strippens vestanden. Möglichkeiten zur Prozessintensivierung beispielsweise durch die Verwendung von Trennwandkolonnen, reaktiven Trennwandkolonnen oder die Möglichkeit der Reaktivdestillation sind dem Fachmann bekannt.

Erfindungsgemäß ist es bevorzugt, dass die Destillation in Verfahrensschritt (ii) bei Drücken von 10 mbar oder kleiner, bevorzugt bei <5 mbar, insbesondere bevorzugt bei 4 mbar bis 0,001 mbar, ganz besonders bevorzugt bei 2 mbar bis 0,01mbar durchgeführt wird. Dies hat den Vorteil, dass die thermische Belastung des Produktes so gering wie möglich gehalten wird und somit unerwünschte Zersetzungs- und Nebenreaktionen minimiert werden können.

Ebenso ist es erfindungsgemäß bevorzugt, dass die Destillation in Verfahrensschritt (ii) bei einer Temperatur von 180 °C bis 280 °C, insbesondere bevorzugt bei 200 °C bis 260 °C, ganz besonders bevorzugt bei 201 °C bis 260 °C durchgeführt wird. Dieser Temperaturbereich ist bevorzugt mit dem oben genannten Druck zu kombinieren, um weiterhin die unerwünschten Zersetzungs- und Nebenreaktionen zu minimieren. Da bei Temperaturen oberhalb von 200 °C für 1,4-Cyclohexandicarbonsäure bereits ein Masseverlust in der Thermogravimetrie beobachtet wurde, war es insbesondere überraschend, dass die erfindungsgemäße Destillation bei diesen Temperaturen mit hohen Ausbeuten und hoher Reinheit verbunden ist.

Erfindungsgemäß werden sowohl die Verfahrensschritte (i) und (ii) bevorzugt in Abwesenheit eines zusätzlichen organischen Lösungsmittels durchgeführt werden. Dies schließt erfindungsgemäß nicht aus, dass sowohl das eingesetzte mindestens eine Carbonat als auch das gebildete Kondensationsprodukt der Reaktion als Lösungsmittel in diesen Reaktionsschritten (soweit möglich) vorhanden sein können. Insbesondere, wenn das Carbonat in einem stöchiometrischem Überschuss zur Dicarbonsäure eingesetzt wird, liegt ein solcher Fall vor. Diese bevorzugte Verfahrensvariante ist besonders schonend. Jedoch ist es erfindungsgemäß bevorzugt, dass kein zusätzliches organisches Lösungsmittel dem Verfahren hinzugefügt wird. Insbesondere bevorzugt werden die Verfahrensschritte (i) und (ii) in Abwesenheit von Methylenchlorid, Methanol und Toluol durchgeführt. Die Abwesenheit eines zusätzlichen organischen Lösungsmittels führt dazu, dass das Verfahren günstig und umweltschonend durchgeführt werden kann.

Das erfindungsgemäße Verfahren liefert einen cycloaliphatischen Diester der Formel (Ia) oder (Ib), 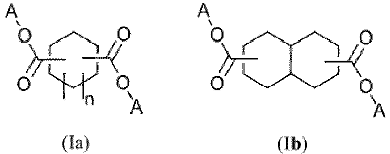 worin
A jeweils unabhängig voneinander für einen aliphatischen oder aromatischen Rest steht und n eine Zahl zwischen 0 und 3 ist, dadurch gekennzeichnet, dass der cycloaliphatische Diester nach dem erfindungsgemäßen Verfahren in sämtlichen oben beschriebenen Ausgestaltungen und Bevorzugungen erhalten wird. Die erfindungsgemäßen Beispiele belegen, dass mit dem nach dem erfindungsgemäßen Verfahren hergestellten cycloaliphatischen Diester andere Polymere mit verbesserten Eigenschaften erhalten werden als mit einem cycloaliphatischen Diester, welcher nicht aufgereinigt wurde. Bei den Vergleichsbeispielen werden Polymere erhalten, die eine intrinsisch schlechtere Farbe aufweisen, selbst nach Ausfällung. Dies bedeutet, dass die entsprechenden Diester ein anderes Nebenproduktspektrum aufweisen als die erfindungsgemäßen Diester. Teile dieser Nebenprodukte der Vergleichsdiester bauen in die Polymerkette ein. Mit den erfindungsgemäßen Diestern konnten dagegen Polymere erhalten werden, welche verbesserte Farbwerte aufweisen, obwohl der Diestern erfindungsgemäß durch die Destillation längeren thermischen Belastungen ausgesetzt wurde. Dies ist insbesondere überraschend, da die Aufreinigungsmethode der Destillation auf dem vorliegenden technischen Gebiet nicht gebräuchlich oder herkömmlich war, da es bekannt war, die cycloaliphatischen Disäuren möglichst wenig thermischem Stress auszusetzten, da ansonsten Zersetzungs- und Nebenproduktreaktionen erwartet werden.

In einem weiteren Aspekt der vorliegenden Erfindung wird ein Verfahren zur Herstellung eines Polyestercarbonats mittels Schmelzeumesterung bereitgestellt, umfassend die Schritte:
(a) Bereitstellung eines cycloaliphatischen Diesters der Formel (Ia) oder (Ib) nach dem erfindungsgemäßen Verfahren in sämtlichen oben beschriebenen Ausgestaltungen und Bevorzugungen und
(b) Reaktion des cycloaliphatischen Diesters der Formel (Ia) oder (Ib) aus Verfahrensschritt (a), mindestens einer Dihydroxy-Verbindung und mindestens eines Diarylcarbonats in einem Schmelzeumesterungsverfahren.

Dabei erfolgt die Reaktion in Verfahrensschritt (b) unter Bedingungen, die geeignet sind, eine Veresterung zu erzielen. Beispielsweise können dem Fachmann bekannte Kondensationskatalysatoren wie beispielsweise Alkalimetallverbindungen, Erdalkalimetallverbindungen, quaternäre Ammoniumverbindungen, quaternäre Phosphoniumverbindungen und beliebige Kombinationen von ihnen, verwendet werden. Ebenso sind die Reaktionsbedingungen der Schmelzeumesterung nicht besonders limitiert, so lange sie geeignet sind, ein entsprechendes Polyestercarbonat herzustellen. Beispielsweise kann die Reaktionstemperatur im Bereich von 100 °C bis 350 °C, bevorzugt 180 °C bis 310 °C liegen. Der Druck kann insbesondere in späteren Verfahrensverlauf reduziert werden. Die Rekationszeit kann zwischen 1 h bis 10 h variieren. Dabei kann die Polymerisation in einer oder mehreren Stufen, wie aus dem Stand der Technik bekannt, durchgeführt werden.

Wie bereits schon oben beschrieben, wurden über dieses erfindungsgemäße Verfahren zur Herstellung eines Polyestercarbonats überraschenderweise Polymere erhalten, welche eine gute intrinsische Eigenfarbe aufweisen.

Insbesondere ist es erfindungsgemäß vorteilhaft, wenn Verfahrensschritt (b) unmittelbar nach Verfahrensschritt (a) erfolgt. Dabei ist der Ausdruck "unmittelbar" so zu verstehen, dass nach der Herstellung des erfindungsgemäßen Diesters und dessen Destillation das aus der Destillation erhaltene Produkt in das Schmelzeumesterungsverfahren gegeben wird. Dadurch muss das Produkt gegebenenfalls nur teilweise kondensiert werden oder ihm die Wärmeenergie nur teilweise entzogen werden, da im Schmelzeumesterungsverfahren ein aufgeschmolzenes Produkt benötigt wird. Die Wärmeenergie muss somit nicht doppelt aufgebracht werden. Ebenso ist es möglich, die bei der Kondensation des destillierten Produktes angefallene Wärme dem Schmelzeumesterungsverfahren über geeignete Mittel zuzuführen, um somit ein wirtschaftliches und umweltfreundliches Verfahren auszulegen.

Die mindestens eine Dihydroxyverbindung in Verfahrensschritt (b) wird bevorzugt ausgewählt aus der Gruppe, bestehend aus 1,2-Cyclohexandiol, 1,3-Cyclohexandiol, 1,4-Cyclohexandiol, 1,2-Cyclohexandimethanol, 1,3-Cyclohexandimethanol, 1,4-Cyclohexandimethanol, Tricyclodecandimethanol, 3,9-bis(1,1-Dimethyl-2-hydroxyethyl)-2,4,8,10-tetraoxaspiro[5.5]undecane, 2,2-bis(4-Hydroxycyclohexyl)propan, Tetrahydro-2,5-furandimethanol, Bisphenol A, 1,1-Bis-(4-hydroxyphenyl)-3,3,5-trimethylcyclohexan, 4,4'-Dihydroxybiphenyl (DOD), 4,4`-Dihydroxybiphenylether (DOD-Ether), Bisphenol B, Bisphenol M, den Bisphenolen (I) bis (III) 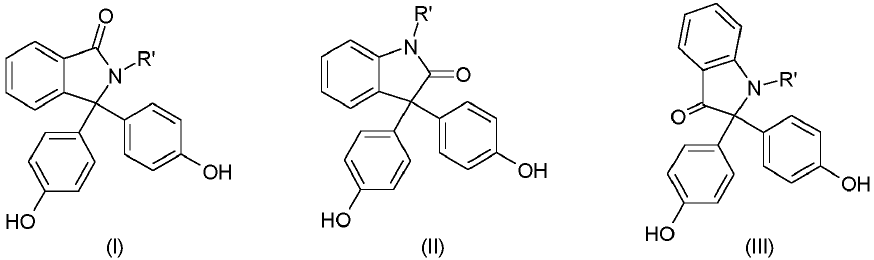 wobei in den Formeln (I) bis (III) R' jeweils für C₁-C₄-Alkyl, Aralkyl oder Aryl, bevorzugt für Methyl oder Phenyl, ganz besonders bevorzugt für Methyl, steht,

Butandiol, Bernsteinsäure, Adipinsäure, Ethylenglykol, Milchsäure, Hexandiol und 1,4:3,6-Dianhydrohexitolen wie Isomannid, Isoidid und Isosorbid. Diese sind auch bekannt als 1,4:3,6-Dianhydro-D-glycidol, 1,4:3,6-Dianhydro-L-iditol und 1,4:3,6-Dianhydro-D-mannitol. Besonders bevorzugt sind dabei aliphatische Dihydroxyverbindungen. Dabei kann in einer Ausfuhrungsform zusätzlich zu der oder den aliphatischen Dihydroxyverbindungen eine aromatische Dihydroxyverbindung im molaren Unterschuss verwendet werden.

Ganz besonders bevorzugt ist die Dihydroxyverbindung ein 1,4:3,6-Dianhydrohexitolen wie Isomannid, Isoidid und Isosorbid und davon am bevorzugsten Isosorbid. Es können auch beliebige Mischungen eingesetzt werden. Ebenso bevorzugt ist die Dihydroxyverbindung eine Mischung aus 1,4:3,6-Dianhydrohexitolen wie Isomannid, Isoidid und Isosorbid und mindestens einem aus der Gruppe aus 1,2-Cyclohexandimethanol, 1,3-Cyclohexandimethanol und/oder 1,4-Cyclohexandimethanol. Dabei ist es insbesondere bevorzugt, dass das 1,2-Cyclohexandimethanol, 1,3-Cyclohexandimethanol und/oder 1,4-Cyclohexandimethanol in Mengen bis zu 20 %, bevorzugt 1 bis 10 %, bezogen auf die gesamte Masse der Dihydroxyverbindungen eingesetzt wird.

Ebenso ist es bevorzugt, dass als Diarylcarbonat in Verfahrensschritt (b) eine Verbindung der Formel (2) eingesetzt wird 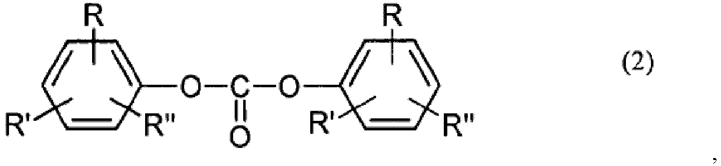 wobei R, R' und R" jeweils unabhängig voneinander gleich oder verschieden sein können und für Wasserstoff, gegebenenfalls verzweigtes C1-C34Alkyl, C7-C34-Alkylaryl oder C6-C34-Aryl stehen, R weiterhin auch -COO-R‴ bedeuten kann, wobei R‴ für gegebenenfalls verzweigtes C1-C34-Alkyl, C7-C34-Alkylaryl oder C6-C34-Aryl steht. Hier gelten ebenso die bereits zu Formel (2) beschriebenen Bevorzugungen. Dabei hat es sich als vorteilhaft herausgestellt, den erfindungsgemäßen cycloaliphatischen Diester mit dem Carbonat - beispielsweise DPC - herzustellen, mit dem man auch in Verfahrensschritt (b) das Polymer herstellt. In Verfahrensschritt (b) stören dann geringe Spuren des beispielhaften DPCs, da dieses dann in Verfahrensschritt (b) ohnehin zugesetzt wird. Damit ist diese Herstellmethode mit DPC sehr vorteilhaft. Andernfalls müssen alle Reagenzien und Nebenprodukte vor der Polymerisation sehr genau entfernt werden.

Daher kann der cycloaliphatische Diester der Formel (Ia) oder (Ib) , 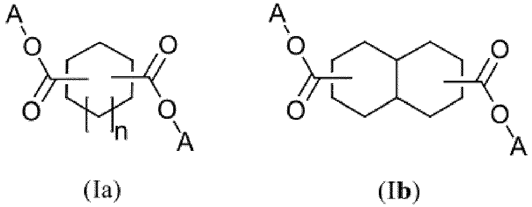 worin A und n die oben beschriebenen Bedeutungen in sämtlichen Kombinationen und Bevorzugungen haben,
auch zur Herstellung eines Polyestercarbonats mittels Schmelzeumesterung durch die Reaktion des cycloaliphatischen Diesters mit einer biobasierten Diydroxyverbindung, bevorzugt ein 1,4:3,6-Dianhydrohexitolen wie Isomannid, Isoidid und Isosorbid, und einem Diarylcarbonat verwendet werden, wobei der cycloaliphatischen Diesters der Formel (Ia) oder (Ib) erhalten wird durch die Reaktion mindestens einer cycloaliphatischen Dicarbonsäure und mindestens eines aliphatischen und/oder aromatischen Carbonats in Anwesenheit eines basischen Katalysators. Hierbei gelten bevorzugt die zum erfindungsgemäßen Verfahren beschriebenen Ausgestaltungen und Bevorzugungen in sämtlichen Kombinationen.

In einem weiteren Aspekt der Erfindung wird ein Polyestercarbonat bereitgestellt, welches erhalten wird nach dem erfindungsgemäßen Verfahren zur Herstellung eines Polyestercarbonats mittels Schmelzeumesterung Verfahren in sämtlichen oben beschriebenen Ausgestaltungen und Bevorzugungen. Die erfindungsgemäßen Polymere weisen überraschenderweise einen niedrigeren Gehalt ein eingebauten farbgebenden Verunreinigungen auf.

### Beispiele:

### Verwendete Materialien:

Cyclohexandicarbonsäure: 1,4-Cyclohexandicarbonsäure; CAS 1076-97-7 99 %; Sigma-Aldrich, München Deutschland
Diphenylcarbonat: Diphenylcarbonat, 99,5 %, CAS 102-09-0; Acros Organics, Geel, Belgien Natriumphenolat: Natriumphenolat -Trihydrat, 98%, CAS 652-67-5; Merck, Darmstadt Deutschland
Tetraphenylphosphonium-Phenolat: Tetraphenylphosphonium-Phenolat, 66,5%, CAS 15464-47-8; Rheinchemie
Tetramethylammoniumhydroxid: Tetramethylammoniumhydroxid Pentahydrat; ≥97% (CAS: 10424-65-4); Sigma-Aldrich, München Deutschland
4-Dimethylaminopyridin: 4-(Dimethylaminopyridin; ≥98,0 %; purum; CAS 1122-58-3; Sigma-Aldrich, München Deutschland
Isosorbid: Isosorbid (CAS: 652-67-5), 98 %, Sigma-Aldrich, München Deutschland; das Isosorbid wird vor Gebrauch aus Isopropanol umkristallisiert.
Irganox^{®} B900 (Hersteller: BASF)

### Analytische Methoden:

### Bestimmung der Glastemperatur:

Die Glastemperatur wird mittels dynamischer Differenzkalorimetrie (DSC) gemäß der Norm DIN EN ISO 11357-1:2009-10 und ISO 11357-2:2013-05 bei einer Heizrate von 10 K/min unter Stickstoff mit Bestimmung der Glastemperatur (Tg) gemessen als Wendepunkt im zweiten Aufheizvorgang bestimmt.

### Chemische Charakterisierung:

¹H-NMR: 600 MHz; Bruker AV III HD 600 Spektrometer; Lösungsmittel: CDCl₃

Das Molekulargewicht (Mn) der Polymerproben wird über das NMR Spektrum (über den Phenylendgruppengehalt) abgeschätzt.

### Messung des Gelbwerts (YI):

Zur Messung des Gelbwerts wurde eine Lösung des entsprechenden Polymers bzw. Edukts in Dichlormethan angesetzt (Dichlormethan; Uvasol der Firma Merck). Dazu wurde eine Konzentration von 0,02 g Polymer bzw. Edukt / ml verwendet. Die jeweilige Lösung wurde in eine Glasküvette (1 cm Schichtdicke) gefüllt. Die Küvette wurde im Shimadzu UV1800 in Transmission vermessen und der Gelbwert (YI) für Lichtart D 65 und 10° Normalbeobachter über die Messung der Farbkoordinaten (CIE) bestimmt und nach ASTM E313-10 berechnet. Dabei wurde der Messwert des reinen Lösungsmittels von den Messergebnissen der jeweiligen Proben abgezogen.

### Beispiel 1

3,44 g (0,0199 Mol) 1,4-Cyclohexandicarbonsäure und 8,99 g (0,0419 Mol) Diphenylcarbonat sowie 0,0031 g (0,0000182 Mol) Natriumphenolat-Trihydrat wurden in einem Kolben mit Kurzwegabscheider vorgelegt. Das Gemisch wurde durch 4-faches evakuieren und belüften mit Stickstoff von Sauerstoff befreit. Das Gemisch wurde aufgeschmolzen und auf 280 °C unter Rühren erwärmt. Der Druck wurde auf Normaldruck belassen. Dass Gemisch wurde für 5 Stunden gerührt und hierbei kontinuierlich Phenol abdestilliert.

Rohprodukt 5,9 g braunes Harz; NMR (1-H-NMR) belegte den Erhalt der gewünschten Verbindung.

Das Beispiel zeigt, dass prinzipiell auch bei höheren Temperaturen gearbeitet werden kann. Allerdings ist es bevorzugt, unter Vakuum und bei tieferer Temperatur zu arbeiten, da die Reaktion schonender verläuft.

### Beispiel 2

3,44 g (0,0199 Mol) 1,4-Cyclohexandicarbonsäure und 8,99 g (0,0419 Mol) Diphenylcarbonat wurden in einem Kolben mit Kurzwegabscheider vorgelegt. Das Gemisch wurde durch 4-faches evakuieren und belüften mit Stickstoff von Sauerstoff befreit. Das Gemisch wurde aufgeschmolzen und auf 160 °C unter Rühren erwärmt. Der Druck wurde auf Umgebungsdruck eingestellt. Dass Gemisch wird für 30 Minuten gerührt; danach wird der Druck auf 10 mbar reduziert. Es wurde für 30 Minuten kontinuierlich abdestilliert, wobei der Druck in dieser Zeit auf 0,5 mbar gesenkt wurde.

| | |
|---|---|
| Sumpfprodukt: | 3,9 g |
| Dest.Vorlage: | 6,4 g |

Die DC-Analyse zeigte, dass es sich beim Sumpfprodukt ausschließlich um Diphenylcarbonat und Cyclohexandicarbonsäure handelt. Beim Destillationsprodukt handelte es sich ausschließlich um Diphenylcarbonat. Damit konnte gezeigt werden, dass sich unter den gewählten Bedingungen nicht das gewünschte Produkt erhalten lässt.

### Beispiel 3

20 g (0,116 Mol) 1,4-Cyclohexandicarbonsäure und 52,18 g (0,243 Mol) Diphenylcarbonat sowie 0,018 g (1,06* 10-4 Mol) Natriumphenolat-Trihydrat und 0,0072 g (0,01 Gew.-% bezogen auf die Summe von DPC und aliphatischer Disäure) Irganox B900 wurden in einem Kolben mit Kurzwegabscheider und Vakuumhahnvorstoß vorgelegt. Das Gemisch wurde durch 4-faches evakuieren und belüften mit Stickstoff von Sauerstoff befreit. Das Gemisch wurde aufgeschmolzen und auf 220 °C unter Rühren erwärmt und fraktioniert destilliert. Der Druck wurde auf 600 mbar eingestellt. Dass Gemisch wurde für 60 Minuten gerührt; danach wurde der Druck bis auf etwa 4 mbar reduziert (Fraktion 1 und 2 wurden abgenommen).

Fraktion 3 wurde zwischen 4,0 und 1,6 mbar entnommen und die 4. Fraktion wurde bei 240°C im Bereich von 1,5 bis 0,9 mbar abdestilliert.

| | |
|---|---|
| Sumpfrückstand: | 5,9 g |
| Fraktion 1: | 15,3 g |
| Fraktion 2: | 7,1 g |
| Fraktion 3: | 6,0 g |
| Fraktion 4: | 23,5 g (62 % d. th. Ausbeute); F. 3 + 4 (78 % d. th. Ausbeute) |

Die DC zeigte, dass bei Fraktion 1 und 2 ausschließlich Phenol abdestilliert wurde.

Fraktion 3 enthielt das Reaktionsprodukt und Spuren von sowohl DPC und Phenol.

Das gewünschte Produkt konnte in hoher Reinheit in Fraktion 4 erhalten werden. Es wurde ein weißer durchsichtiger Feststoff erhalten.

### Beispiel 4

3,44 g (0,0199 Mol) 1,4-Cyclohexandicarbonsäure und 8,99 g (0,0419 Mol) Diphenylcarbonat wurden in einem Kolben mit Kurzwegabscheider vorgelegt. Das Gemisch wurde durch 4-faches evakuieren und belüften mit Stickstoff von Sauerstoff befreit. Das Gemisch wurde aufgeschmolzen und auf 180 °C unter Rühren erwärmt. Der Druck wurde auf 600 mbar eingestellt. Dass Gemisch wurde für 60 Minuten gerührt; danach wurde der Druck auf 10 mbar reduziert. Es wurde für 30 Minuten kontinuierlich DPC abdestilliert, wobei der Druck in dieser Zeit auf 5 mbar gesenkt wurde.

| | |
|---|---|
| Sumpfrückstand: | 4,67 g |
| Dest. Vorlage: | 8,0 g |

Die DC zeigte, dass ausschließlich DPC abdestilliert wurde, das gewünschte Produkt konnte nicht erhalten werden.

### Beispiel 5

34,4 g (0,199 Mol) 1,4-Cyclohexandicarbonsäure und 89,9 g (0,419 Mol) Diphenylcarbonat sowie 0,031 g (0,00027 Mol) Natriumphenolat-Trihydrat wurden in einem Kolben mit Kurzwegabscheider vorgelegt. Das Gemisch wurde durch 4-faches evakuieren und belüften mit Stickstoff von Sauerstoff befreit. Das Gemisch wurde aufgeschmolzen und auf 230 °C unter Rühren erwärmt. Dass Gemisch wurde unter Normaldruck bei 230 °C für 3 Stunden gerührt; dabei destillierte Phenol ab. Danach wurde die Temperatur der Reaktionsschmelze auf 200 °C reduziert und der Druck auf 500 mbar reduziert. Dabei destillierte weiterhin Phenol ab. Das Gemisch wurde für eine Stunde gerührt und der Druck dabei kontinuierlich bis auf 5 mbar abgesenkt. Die flüchtigen Bestandteile wurden kontinuierlich entfernt. Die

Temperatur wurde auf 210 °C angehoben und das Vakuum auf 1 mbar reduziert. Es wurde solange destilliert bis keine flüchtigen Bestandteile mehr abgezogen wurden und die Kopftemperatur deutlich fiel. Die Destillationsvorlage wurde gewechselt, die Temperatur auf 220 °C angehoben und das Vakuum auf 0,5 mbar reduziert. Dabei destillierte das Produkt. Es wurde für 30 Minuten destilliert; die Kopftemperatur stieg dabei auf 180 - 190 °C. Man erhielt ein helles weißes Produkt. Ausbeute: 59,7 g (91,5 % d. Th.).

### Beispiel 6

### Versuch in Anlehnung an Beispiel 2 der JP H07-126213

16,6 g (0,096 mol) 1,4-Cyclohexandicarbonsäure, 45,0 g (0,21 mol) Diphenylcarbonat sowie 0,09 g 4-Dimethylaminopyridin wurden in einem Dreihalskolben mit Kurzwegabscheider vorgelegt. Die Apparatur wurde durch vierfaches evakuieren und belüften mit Stickstoff von Sauerstoff befreit. Das Gemisch wurde bei 180 °C aufgeschmolzen und auf 220 °C unter Rühren erwärmt. Der Druck wurde innerhalb von 45 Minuten auf 700 mbar reduziert, und dann innerhalb von weiteren 45 Minuten auf 2 mbar reduziert. Dabei wurde kontinuierlich Phenol entfernt.

Das Produkt wurde entnommen. Man erhält ein grau-weißes Pulver. Die NMR-Analyse zeigte das gewünschte Produkt. Ausbeute : 17,5 g. Das Produkt wurde ohne Reinigung weiter eingesetzt.

### Beispiel 7

### Versuch in Anlehnung an Beispiel 4 der WO0210111

4,65 g (0,027 mol) 1,4-Cyclohexandicarbonsäure, 17,6 g (0,082 mol) Diphenylcarbonat sowie 0,3 g einer 24 %igen Lösung von Tetramethylammoniumhydroxidlösung in Wasser (ca. 0,0008 mol) wurden in einem Dreihalskolben mit Kurzwegabscheider vorgelegt. Die Apparatur wurde durch vierfaches evakuieren und belüften mit Stickstoff von Sauerstoff befreit. Das Gemisch wurde bei 180 °C aufgeschmolzen und auf 210 °C unter Rühren erwärmt. Das Reaktionsgemisch wurde für 4,5 Stunden bei dieser Temperatur gerührt. Dann wurde die Temperatur auf 150 °C gesenkt und der Druck auf 2 mbar gesenkt. Es wurde für eine Stunde gerührt und kontinuierlich Phenol entfernt.

Das Produkt wurde entnommen. Man erhielt ein grau-beiges Pulver. Das NMR (1H-NMR) belegte das gewünschte Produkt. Ausbeute: 20,3 g

### Beispiel 8

32,2 g (0,187 mol) 1,4-Cyclohexandicarbonsäure, 210,0 g (0,980 mol) Diphenylcarbonat sowie 0,18 g 4-Dimethylaminopyridin wurden in einem Dreihalskolben mit Kurzwegabscheider vorgelegt. Die Apparatur wurde durch vierfaches evakuieren und belüften mit Stickstoff von Sauerstoff befreit. Das Gemisch wurde bei 180 °C aufgeschmolzen und auf 220 °C unter Rühren erwärmt. Der Druck wurde innerhalb von ca. 60 Minuten auf 700 mbar reduziert, und dann innerhalb von weiteren 4 Stunden auf 10 mbar reduziert. Dabei wurde kontinuierlich Phenol entfernt. Der Druck wurde innerhalb von 50 Minuten auf 15 mbar reduziert; dabei wurden flüchtige Bestandteile entfernt. Der Druck wurde innerhalb von 30 Minuten auf 1,5 mbar reduziert.

Zur Destillation des Produktes wurde die Temperatur auf 210 bis 215 °C gesenkt und der Druck auf 1 mbar gesenkt. Bei diesen Bedingungen wurde die Produktfraktion abgenommen.

Das Produkt wurde entnommen. Man erhielt einen weißen Feststoff (Ausbeute 43 g; 71 % d. Th.).

**Tabelle 1: Optische Daten (YI)**

| Produkt aus Beispiel | YI |
|---|---|
| 5 | 0,07 |
| 6 | 17,67 |
| 7 | 1,18 |
| 8 | 0,0 |

Die optischen Daten zeigen, dass die Destillation der jeweiligen Produkte zu einem Diester mit besserer optischer Qualität führen.

### Polymerisation von Cyclohexandicarbonsäurediphenylester mit Isosorbid.

### Beispiel A

8,00 g (0,0246 mol) 1,4-Cyclohexandicarbonsäurediphenylester aus Beispiel 5und 8,98 g (0,0616 mol) Isosorbid sowie 8,30 g (0,0387 mol) Diphenylcarbonat wurden in einem Kolben mit Kurzwegabscheider vorgelegt. Es wurden 50 ppm Cäsiumcarbonat sowie 50 ppm Natriumhydroxid (wässrige Lösung von Natriumhydroxid in Wasser; 6,5 %) zugegeben. Der Kolben wurde durch 4-faches evakuieren und belüften mit Stickstoff von

Sauerstoff befreit. Das Reaktionsgemisch wurde bei 190 °C aufgeschmolzen und der Druck vorsichtig innerhalb von ca. 15 Minuten auf 200 mbar reduziert. Das Reaktionsgemisch wurde für 20 Minuten gerührt. Der Druck wurde auf 100 mbar reduziert und die Temperatur auf 210 °C erhöht. Es wurde für 30 Minuten bei diesen Bedingungen gerührt, wobei Phenol kontinuierlich aus dem Reaktionsgemisch entfernt wurde. Der Druck wurde auf 10 mbar reduziert und nach weiteren 10 Minuten auf 2 mbar reduziert. Bei 2 mbar wurde noch 15 Minuten gerührt. Danach wurde das Produkt entnommen, in Dichlormethan gelöst (das Produkt war vollständig in Dichlormethan löslich) und in Methanol gefällt. Man erhielt ein weißes Pulver.

### Beispiel B

Das Verfahren wurde wie in Beispiel A durchgeführt. Als Edukt wurde das Produkt aus Beispiel 8 eingesetzt.

### Beispiel C

Das Verfahren wurde wie in Beispiel A durchgeführt. Als Edukt wurde das Produkt aus Beispiel 7 eingesetzt.

### Beispiel D

Das Verfahren wurde wie in Beispiel A durchgeführt. Als Edukt wurde das Produkt aus Beispiel 6 eingesetzt.

**Tabelle 2: Optische Messung (nach Fällung)**

| Beispiel | Tg [°C] | Mn (NMR) [g/mol] | YI |
|---|---|---|---|
| A | 131 | Ca. 5000 | 7,9 |
| B | 141 | Ca. 11000 | 7,06 |
| C | 131 | Ca. 7000 | 12,4 |
| D | 135 | Ca. 5700 | 19,8 |

Die Helligkeit und intrinsische Eigenfarbe sind ein wesentliches Qualitätsmerkmal von Polymeren. Die Eigenfarbe der Polymere wird normalerweise durch niedermolekulare Produkte verursacht. Diese Produkte entstehen bei der Polykondensation und bauen in der Regel nicht in die Polymerkette ein. Durch Fällung des Polymers lassen sich diese Verunreinigungen in der Regel entfernen. Allerdings zeigte sich überraschenderweise, dass die über Destillation aufgereinigten Produkte nach Fällung eine geringere Eigenfarbe des Polymers zur Folge haben. Dies bedeutet, dass bei den nicht destillierten Vergleichsproben ein Einbau der farbgebenden Produkte in die Polymerkette stattgefunden hat, wohingegen im den erfindungsgemäßen Diarylestern ein solcher Einbau reduziert werden kann.

## Patentansprüche

1. Verfahren zur Herstellung eines cycloaliphatischen Diesters der Formel (Ia) oder (Ib), 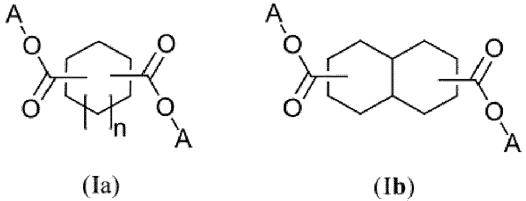 worin
A jeweils unabhängig voneinander für einen aliphatischen oder aromatischen Rest steht
und
n eine Zahl zwischen 0 und 3 ist,
umfassend die Schritte
(i) Reaktion eines Gemisches, umfassend mindestens eine cycloaliphatische Dicarbonsäure und mindestens ein aliphatisches und/oder aromatisches Carbonat, in Anwesenheit eines basischen Katalysators zu einem cycloaliphatischen Diester der Formeln (Ia) oder (Ib) und
(ii) Abtrennung des cycloaliphatischen Diesters der Formel (Ia) oder (Ib) vom Gemisch des Verfahrensschritts (i) mittels Destillation bei einer Temperatur von 180°C bis 280°C.

2. Verfahren nach Anspruch 1, **dadurch gekennzeichnet, dass** die Destillation in Verfahrensschritt (ii) bei Drücken von 10 mbar oder kleiner durchgeführt wird.

3. Verfahren nach einem der Ansprüche 1 oder 2, **dadurch gekennzeichnet, dass** in Verfahrensschritt (i) ein aromatisches Carbonat der Formel (2) eingesetzt wird 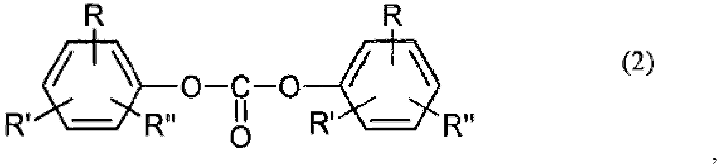 wobei R, R' und R" jeweils unabhängig voneinander gleich oder verschieden sein können und für Wasserstoff, gegebenenfalls verzweigtes C1-C34Alkyl, C7-C34-Alkylaryl oder C6-C34-Aryl stehen, R weiterhin auch -COO-R‴ bedeuten kann, wobei R‴ für gegebenenfalls verzweigtes C1-C34-Alkyl, C7-C34-Alkylaryl oder C6-C34-Aryl steht.

4. Verfahren nach Anspruch 3, **dadurch gekennzeichnet, dass** in Verfahrensschritt (i) Diphenylcarbonat als aromatisches Carbonat eingesetzt wird.

5. Verfahren nach einem der Ansprüche 1 bis 4, **dadurch gekennzeichnet, dass** Verfahrensschritte (i) und (ii) in Abwesenheit eines zusätzlichen organischen Lösungsmittels durchgeführt werden.

6. Verfahren gemäß einem der Ansprüche 1 bis 5, **dadurch gekennzeichnet, dass** im Verfahrensschritt (i) mindestens ein Stabilisator ausgewählt aus der Gruppe bestehend aus P-haltigen Stabilisatoren und/oder phenolischen Radikalfängern zugesetzt wird.

7. Verfahren nach einem der Ansprüche 1 bis 6, **dadurch gekennzeichnet, dass** die Reaktion des Verfahrensschritts (i) bei Temperaturen von 180 bis 280 °C durchgeführt wird.

8. Verfahren nach einem der Ansprüche 1 bis 7, **dadurch gekennzeichnet, dass** während der Reaktion in Verfahrensschritt (i) die flüchtigen Bestandteile, welche einen Siedepunkt unterhalb des cycloaliphatischen Diesters der Formel (Ia) oder (Ib) und unterhalb des aliphatischen und/oder aromatischen Carbonats aufweisen, mittels Destillation gegebenenfalls schrittweise abgetrennt werden.

9. Verfahren zur Herstellung eines Polyestercarbonats mittels Schmelzeumesterung, umfassend die Schritte:
(a) Bereitstellung eines cycloaliphatischen Diesters der Formel (Ia) oder (Ib) gemäß dem Verfahren nach einem der Ansprüche 1 bis 8 und
(b) Reaktion des cycloaliphatischen Diesters der Formel (Ia) oder (Ib) aus Verfahrensschritt (a), mindestens einer Dihydroxy-Verbindung und mindestens eines Diarylcarbonats in einem Schmelzeumesterungsverfahren.

10. Verfahren nach Anspruch 9, **dadurch gekennzeichnet, dass** Verfahrensschritt (b) unmittelbar nach Verfahrensschritt (a) erfolgt.

11. Verfahren nach Anspruch 9 oder 10, **dadurch gekennzeichnet, dass** die Dihydroxyverbindung in Verfahrenschritt (b) ausgewählt ist aus der Gruppe, bestehend aus 1,2-Cyclohexandiol, 1,3-Cyclohexandiol, 1,4-Cyclohexandiol, 1,2-Cyclohexandimethanol, 1,3-Cyclohexandimethanol, 1,4-Cyclohexandimethanol, Tricyclodecandimethanol, 3,9-bis(1,1-Dimethyl-2-hydroxyethyl)-2,4,8,10-tetraoxaspiro[5.5]undecane, 2,2-bis(4-Hydroxycyclohexyl)propan, Tetrahydro-2,5-furandimethanol, Bisphenol A, 1,1-Bis-(4-hydroxyphenyl)-3,3,5-trimethylcyclohexan, 4,4'-Dihydroxybiphenyl (DOD), 4,4'-Dihydroxybiphenylether (DOD-Ether), Bisphenol B, Bisphenol M, den Bisphenolen (I) bis (III) 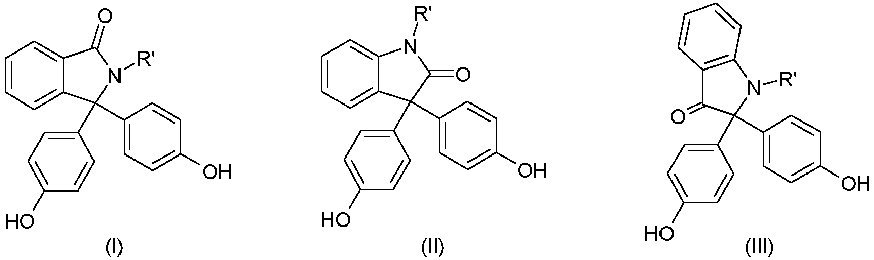 wobei in den Formeln (I) bis (III) R' jeweils für C₁-C₄-Alkyl, Aralkyl oder Aryl, bevorzugt für Methyl oder Phenyl, ganz besonders bevorzugt für Methyl, steht,
Butandiol, Bernsteinsäure, Adipinsäure, Ethylenglykol, Milchsäure, Hexandiol und 1,4:3,6-Dianhydrohexitolen wie Isomannid, Isoidid und Isosorbid.

12. Verfahren nach einem der Ansprüche 9 bis 11, **dadurch gekennzeichnet, dass** als Diarylcarbonat in Verfahrensschritt (b) eine Verbindung der Formel (2) eingesetzt wird 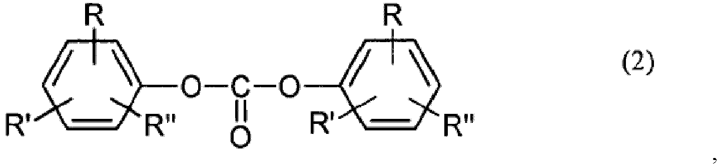 wobei R, R' und R" jeweils unabhängig voneinander gleich oder verschieden sein können und für Wasserstoff, gegebenenfalls verzweigtes C1-C34Alkyl, C7-C34-Alkylaryl oder C6-C34-Aryl stehen, R weiterhin auch -COO-R‴ bedeuten kann, wobei R‴ für gegebenenfalls verzweigtes C1-C34-Alkyl, C7-C34-Alkylaryl oder C6-C34-Aryl steht.

13. Polyestercarbonat, erhalten nach dem Verfahren nach einem der Ansprüche 9 bis 12.

## Claims

1. Method for producing a cycloaliphatic diester of formula (Ia) or (Ib), 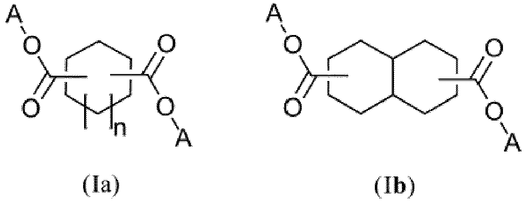 in which
A each independently is an aliphatic or aromatic radical
and
n is a number between 0 and 3,
comprising the steps of
(i) reacting a mixture comprising at least one cycloaliphatic dicarboxylic acid and at least one aliphatic and/or aromatic carbonate, in the presence of a basic catalyst, to give a cycloaliphatic diester of formulae (Ia) or (Ib) and
(ii) separating the cycloaliphatic diester of formula (Ia) or (Ib) from the mixture of method step (i) by means of distillation at a temperature of 180°C to 280°C.

2. Method according to Claim 1, **characterized in that** the distillation in method step (ii) is conducted at pressures of 10 mbar or lower.

3. Method according to either of Claims 1 and 2, **characterized in that**, in method step (i), an aromatic carbonate of formula (2) is used 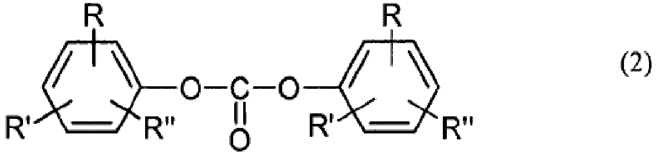 , where R, R' and R" each independently can be the same or different and are hydrogen, optionally branched C1-C34-alkyl, C7-C34-alkylaryl or C6-C34-aryl, R can additionally also denote -COO-R'", where R'" is optionally branched C1-C34-alkyl, C7-C34-alkylaryl or C6-C34-aryl.

4. Method according to Claim 3, **characterized in that**, in method step (i), diphenyl carbonate is used as aromatic carbonate.

5. Method according to any of Claims 1 to 4, **characterized in that** method steps (i) and (ii) are conducted in the absence of an additional organic solvent.

6. Method according to any of Claims 1 to 5, **characterized in that**, in method step (i), at least one stabilizer selected from the group consisting of P-containing stabilizers and/or phenolic free-radical scavengers is added.

7. Method according to any of Claims 1 to 6, **characterized in that** the reaction of method step (i) is conducted at temperatures of 180 to 280°C.

8. Method according to any of Claims 1 to 7, **characterized in that**, during the reaction in method step (i), the volatile constituents which have a boiling point below the cycloaliphatic diester of formula (Ia) or (Ib) and below the aliphatic and/or aromatic carbonate are removed, optionally in steps, by means of distillation.

9. Method for producing a polyestercarbonate by means of melt transesterification, comprising the steps of:
(a) providing a cycloaliphatic diester of formula (Ia) or (Ib) by the method according to any of Claims 1 to 8 and
(b) reacting the cycloaliphatic diester of formula (Ia) or (Ib) from method step (a), at least one dihydroxy compound and at least one diaryl carbonate in a melt transesterification process.

10. Method according to Claim 9, **characterized in that** method step (b) is performed immediately after method step (a).

11. Method according to Claim 9 or 10, **characterized in that** the dihydroxy compound in method step (b) is selected from the group consisting of cyclohexane-1,2-diol, cyclohexane-1,3-diol, cyclohexane-1,4-diol, cyclohexane-1,2-dimethanol, cyclohexane-1,3-dimethanol, cyclohexane-1,4-dimethanol, tricyclodecanedimethanol, 3,9-bis(1,1-dimethyl-2-hydroxyethyl)-2,4,8,10-tetraoxaspiro[5.5]undecane, 2,2-bis(4-hydroxycyclohexyl)propane, tetrahydro-2,5-furandimethanol, bisphenol A, 1,1-bis(4-hydroxyphenyl)-3,3,5-trimethylcyclohexane, 4,4'-dihydroxybiphenyl (DOD), 4,4'-dihydroxybiphenyl ether (DOD ether), bisphenol B, bisphenol M, the bisphenols (I) to (III) 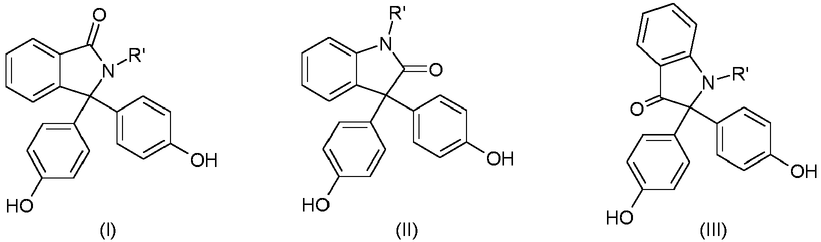 where in the formulae (I) to (III) R' in each case is C₁-C₄-alkyl, aralkyl or aryl, preferably methyl or phenyl, very particularly preferably methyl, butanediol, succinic acid, adipic acid, ethylene glycol, lactic acid, hexanediol and 1,4:3,6-dianhydrohexitols such as isomannide, isoidide and isosorbide.

12. Method according to any of Claims 9 to 11, **characterized in that** as diaryl carbonate in method step (b) a compound of formula (2) is used 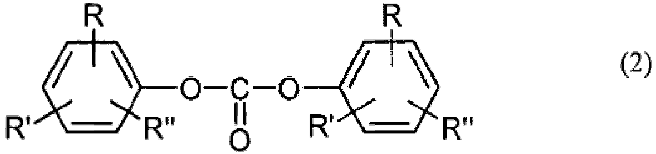 , where R, R' and R" each independently can be the same or different and are hydrogen, optionally branched C1-C34-alkyl, C7-C34-alkylaryl or C6-C34-aryl, R can additionally also denote -COO-R'", where R'" is optionally branched C1-C34-alkyl, C7-C34-alkylaryl or C6-C34-aryl.

13. Polyestercarbonate, obtained by the method according to any of Claims 9 to 12.

## Revendications

1. Procédé de production d'un diester cycloaliphatique de formule (Ia) ou (Ib), 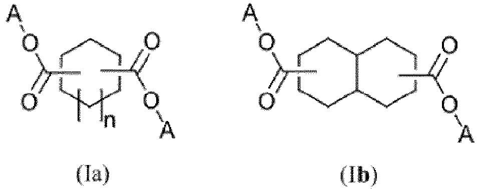 formules dans lesquelles
A représente chaque fois indépendamment les uns des autres un radical aliphatique ou aromatique
et
n est un nombre entre 0 et 3,
comprenant les étapes
(i) réaction d'un mélange, comprenant au moins un acide dicarboxylique cycloaliphatique et au moins un carbonate aliphatique et/ou un carbonate aromatique, en présence d'un catalyseur basique, conduisant à un diester cycloaliphatique de formule (Ia) ou (Ib) et
(ii) séparation du diester cycloaliphatique de formule (Ia) ou (Ib) d'avec le mélange de l'étape (i) du procédé, par distillation à une température de 180 °C à 280 °C.

2. Procédé selon la revendication 1, **caractérisé en ce que** la distillation dans l'étape (ii) du procédé est effectuée sous des pressions de 10 mbars ou moins.

3. Procédé selon l'une quelconque des revendications 1 et 2, **caractérisé en ce que** dans l'étape (i) du procédé est utilisé un carbonate aromatique de formule (2) 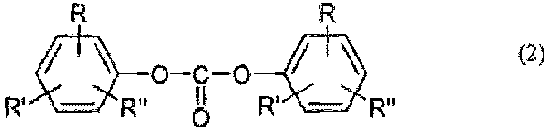 dans laquelle R, R' et R' ' chaque fois indépendamment les uns des autres peuvent être identiques ou différents et représentent un atome d'hydrogène, un groupe alkyle en C₁-C₃₄, alkylaryle en C₇-C₃₄ ou aryle en C₆-C₃₄, éventuellement ramifié, R peut en outre également représenter -COO-R''', R' ' ' représentant un groupe alkyle en C₁-C₃₄, alkylaryle en C₇-C₃₄ ou aryle en C₆-C₃₄, éventuellement ramifié.

4. Procédé selon la revendication 3, **caractérisé en ce que** dans l'étape (i) du carbonate de diphényle est utilisé en tant que carbonate aromatique.

5. Procédé selon l'une quelconque des revendications 1 à 4, **caractérisé en ce que** les étapes (i) et (ii) du procédé sont effectuées en absence d'un solvant organique supplémentaire.

6. Procédé selon l'une quelconque des revendications 1 à 5, **caractérisé en ce que** dans l'étape (i) du procédé est ajouté au moins un stabilisant choisi dans le groupe constitué par les stabilisants contenant du phosphore et/ou les capteurs phénoliques de radicaux.

7. Procédé selon l'une quelconque des revendications 1 à 6, **caractérisé en ce que** la réaction de l'étape (i) du procédé est effectuée à des températures de 180 à 280 °C.

8. Procédé selon l'une quelconque des revendications 1 à 7, **caractérisé en ce que** pendant la réaction dans l'étape (i) du procédé les composés volatils qui présentent un point d'ébullition inférieur à celui du diester cycloaliphatique de formule (Ia) ou (Ib) et inférieur à celui du carbonate aliphatique et/ou du carbonate aromatique sont séparés par distillation éventuellement graduellement.

9. Procédé pour la production d'un polyestercarbonate par transestérification en masse fondue, comprenant les étapes :
(a) préparation d'un diester cycloaliphatique de formule (Ia) ou (Ib) conformément au procédé selon l'une quelconque des revendications 1 à 8 et
(b) réaction du diester cycloaliphatique de formule (Ia) ou (Ib) provenant de l'étape (a) du procédé, d'au moins un composé dihydroxylé et d'au moins un carbonate de diaryle dans un procédé de transestérification en masse fondue.

10. Procédé selon la revendication 9, **caractérisé en ce que** l'étape (b) du procédé s'effectue immédiatement après l'étape (a) du procédé.

11. Procédé selon la revendication 9 ou 10, **caractérisé en ce que** le composé dihydroxylé dans l'étape (b) du procédé est choisi dans le groupe constitué par le 1,2-cyclohexanediol, le 1,3-cyclohexanediol, le 1,4-cyclohexanediol, le 1,2-cyclohexanediméthanol, le 1,3-cyclohexanediméthanol, le 1,4-cyclohexanediméthanol, le tricyclodécanediméthanol, le 3,9-bis(1,1-diméthyl-2-hydroxyéthyl)-2,4,8,10-tétraoxaspiro[5.5]undécane, le 2,2-bis(4-hydroxycyclohexyl)propane, le tétrahydro-2,5-furanediméthanol, le bisphénol A, le 1,1-bis-(4-hydroxyphényl)-3,3,5-triméthylcyclohexane, le 4,4'-dihydroxybiphényle (DOD), un éther de 4,4'-dihydroxybiphényle (éther de DOD), le bisphénol B, le bisphénol M, les bisphénols (I) à (III) 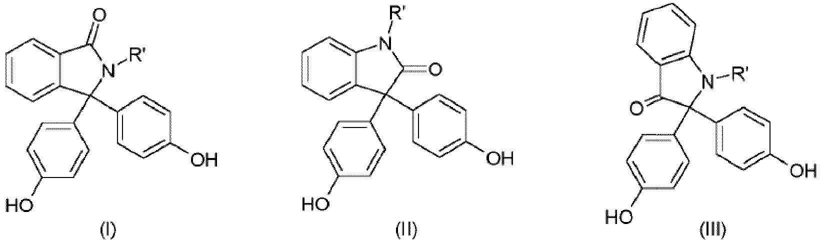 dans les formules (I) à (III) R' représentant chaque fois un groupe alkyle en C₁-C₄, aralkyle ou aryle, de préférence méthyle ou phényle, de façon tout particulièrement préférée méthyle, le butanediol, l'acide succinique, l'acide adipique, l'éthylèneglycol, l'acide lactique, l'hexanediol et les 1,4:3,6-dianhydrohexitols tels que l'isomannide, l'isoidide et l'isosorbide.

12. Procédé selon l'une quelconque des revendications 9 à 11, **caractérisé en ce qu'**en tant que carbonate de diaryle dans l'étape (b) du procédé est utilisé un composé de formule (2) 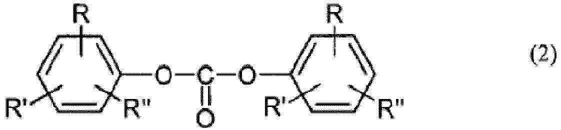 dans laquelle R, R' et R' ' chaque fois indépendamment les uns des autres peuvent être identiques ou différents et représentent un atome d'hydrogène, un groupe alkyle en C₁-C₃₄, alkylaryle en C₇-C₃₄ ou aryle en C₆-C₃₄, éventuellement ramifié, R peut en outre également représenter -COO-R‴, R‴ représentant un groupe alkyle en C₁-C₃₄, alkylaryle en C₇-C₃₄ ou aryle en C₆-C₃₄, éventuellement ramifié.

13. Polyestercarbonate obtenu conformément au procédé selon l'une quelconque des revendications 9 à 12.
